# EUROPEAN PATENT APPLICATION

(11) **EP 4 755 602 A1**
(43) Date of publication of application: **10.06.2026**
(21) Application number: 25218321.5
(22) Date of filing: 25.11.2025
(51) Int. Cl.: B27G 1/00, B08B 1/12, B08B 1/20, B08B 1/34, B08B 1/36

(54) **MACHINING DEVICE AND METHOD OF REMOVING SURFACE AND DEEP DEFECTS IN WOODEN BOARDS**

(30) Priority: 04.12.2024 PL 45045624
(71) Applicant: "Barlinek" Spólka Akcyjna, 25-323 Kielce (PL)
(72) Inventor: FIK, Rafa, 74-320 Barlinek (PL); KUBIAK, Rafa, 74-320 Barlinek (PL)
(74) Representative: BRANDPAT Patent and Trademark Attorneys Chlebicka Czyz Ziolkowski Professional Partnership

(57) **Abstract**

A device has an guide plate (11) disposed under an upper beam (7) of a support structure (2) above the transport assembly (3); a hammer assembly (9) with at least one row of vertical hammer rods (10) loosely seated in the guide plate (11); actuators (8) for displacing the vertical hammer rods (10); retaining elements (12) shaped on the vertical hammer rods (10); a brush cleaning assembly (13) disposed downstream of the hammer assembly (9) in the direction of displacement of the machined wooden boards (6), and a pneumatic cleaning assembly (14) disposed downstream of the brush cleaning assembly (13) in the direction of displacement of the machined wooden boards (6).

The actuators (8) displacing the vertical hammer rods (10) are connected on one side to the guide plate (11) and on the other side to the upper beam (7) of the support structure (2), wherein the vertical hammer rods (10) loosely seated in the guide plate (11) openings (28) have the retaining elements (12) for contacting the guide plate (11).

Surface and deep defects in the wooden boards (6) are removed by lifting the guide plate (11) together with the vertical hammer rods (10), and then lowering this guide plate (11), allowing the vertical hammer rods (10) loosely seated therein to fall gravitationally and causing surface and deep impact contact with the machined wooden boards (6).

## Description

### TECHNICAL FIELD

The subject of the invention is a machining device for removing surface and deep defects in wooden boards and a method of removing surface and deep defects in wooden boards. The invention is particularly applicable to the removal of defects such as loose knots, bark, or wood fibers not permanently bound to the surrounding woody tissue. Machining of semi-finished products is an essential procedure to obtain the necessary surface adhesion for carrying out a repair filling operation of wood tissue decrements of semi-finished products made of hardwood or coniferous wood, intended for the production of floor boards. Wood is a product of plant origin, which causes its structure and properties to depend on many external factors such as soil, climate, mechanical forces and the like. These factors affect a variety of deviations from the normal shape, structure and properties of wood, making it a heterogeneous material, as opposed to materials such as metals, plastics or glass, for example. In the case of wood, it is difficult to obtain a model and repeatable structure. Different types of deviations will always occur because wood does not have a homogeneous anatomical structure. A feature of wood is anisotropy, i.e. the diversity of structure and the occurrence of dimensional changes as a result of sorption or desorption of water under normal changing microclimatic conditions during weather changes and seasons. There are also various decrements and damage to wood in semi-finished products, in terms of their type, size and location, and related to the variable texture of wood, which makes it much more difficult to identify such damage. Loosening and cracking of knots, being the result of drying the wood to obtain the technological value of humidity, are particularly problematic. The knots become loose because they shrink more than the surrounding wood. If the knots have not grown together with the surrounding wood or are partially spoiled, they can loosen or even fall out spontaneously, which results in through holes on the surface of the semi-finished products.

### BACKGROUND ART

Preparation of knot holes and other wood decrements for filling operations is mainly done manually using wire brushes, scrapers and power tools.

There are also known devices and methods for cutting holes in damaged areas of wooden semi-finished products and for fitting accurate wooden discs. A method of repairing defects of various shapes in wood by making a hole in the place of damage and forming a plug filling the hole is known from US5440859.

Also known from the description of the invention BE398694 is a device for repairing knot holes in boards, comprising vertically displaceable drilling devices and drilling devices placed on a laterally slidable carriage. The device further has an axially displaceable plug driving assembly.

A more automated device for repairing defects in wood is known from the description of the invention CN113843866A. In the invention, the repaired board is moved by the transport device to the detection device, where the arrangement and types of defects on the surface of the wooden board are determined. Then the wooden board is moved into the area of the gripping mechanism, after which the transferring mechanism drives properly selected plugs to selected places of the moving wooden board.

From US3516464 there is also known a device and method for removing wood defects, comprising a support structure with a transport assembly for horizontally moving the machined thin veneer panels on the transport rollers. The device is equipped with a hammer assembly with a number of vertical hammer rods slidably guided in the lower guide plate openings and in the upper guide plate openings. The hammer assembly has means for inducing a reciprocating movement of the vertical hammer rods to subject the wood to the forces associated with the impacts of the hammer rods, as a result of which various types of defects, such as knots, are loosened and removed. The hammer rods have, in their central portion, retaining rings cooperating with cams. In addition, two springs are provided on each of the hammer rods, the upper spring being provided between the retaining ring of the hammer rod and the lower spring between the retaining ring and the lower guide plate.

The use of mechanical actuators, and in particular spring drives, may cause damage to the machined surfaces. At some drive frequencies, close to the natural vibration frequencies of the springs used, the springs may fall into a resonance causing an uncontrolled impact force of the hammer rods against the surface of the wooden boards and their damage.

The aim of the invention is to develop solutions enabling cleaning and preparing wood for repair filling operations, without the use of drilling and milling operations, and without driving matching wooden discs into cut holes. Removal of surface or deep defects should be carried out in the least invasive manner, with the greatest possible preservation of the healthy structure of the wood, and thus maintaining its natural appearance.

Another object of the invention is to use such a hammer assembly design for punching loose surface and deep defects of wood that would provide a specific and easily adjustable choice of the impact energy of the hammer rods so as not to cause significant damage to the healthy structure of the wood, and at the same time enable loosening and removal of various types of defects, such as knots, with high efficiency and efficacy.

### DISCLOSURE OF THE INVENTION

The machining device of the invention has a support structure; a transport assembly for horizontal displacement of the machined wooden boards on the transport rollers; a guide plate with openings, provided under an upper beam of the support structure above the transport assembly; a hammer assembly with at least one row of vertical hammer rods loosely seated in the guide plate openings; actuators for displacing the vertical hammer rods; retaining elements shaped on the vertical hammer rods; a brush cleaning assembly disposed downstream of the hammer assembly in the direction of displacement of the machined wooden boards, and a pneumatic cleaning assembly disposed downstream of the brush cleaning assembly in the direction of displacement of the machined wooden boards.

The device according to the invention is characterized in that the actuators displacing the vertical hammer rods are connected on one side to the guide plate and on the other side to the upper beam of the support structure, wherein the vertical hammer rods loosely seated in the guide plate openings have retaining elements for contact with the guide plate.

Preferably, pneumatic actuators are used as the actuators.

Preferably, the retaining elements of the vertical hammer rods are positioned above the guide plate.

Preferably, the retaining elements are formed in the upper face portion of the vertical hammer rods.

Preferably, the retaining elements are formed as rings.

Preferably, the vertical hammer rods have a cutting portion shaped in their lower portion.

Preferably, the cutting portion is formed around the circumference of the lower face portion.

Preferably, the vertical hammer rod has a tapered guiding surface with the lower cutting portion formed around the circumference of the lower face portion and with the upper cutting portion formed at the connection of the tapered guiding surface and the cylindrical guiding portion.

Preferably, the vertical hammer rod has a rasp-cutting portion in the form of rasp teeth notched in the tapered guiding surface, between the lower cutting portion of the lower face portion and the upper cutting portion formed at the connection of the tapered guiding surface and the cylindrical guiding portion.

A method of removing surface and deep defects in wooden boards comprises steps in which:
- machined wooden boards are displaced into the area of operation of the hammer assembly with vertical hammer rods loosely seated in the guide plate openings, placed under the upper beam of the support structure;
- vertical hammer rods are driven into surface and deep impact contact with the machined wooden boards, after which the machined wooden boards are displaced to the operation area of a brush cleaning assembly and a pneumatic cleaning assembly.

The method according to the invention is characterized in that the guide plate is raised together with the vertical hammer rods, and then this guide plate is lowered, allowing the gravitational falling of the vertical hammer rods loosely seated therein for surface and deep impact contact with the machined wooden boards.

Preferably, the guide plate is raised and lowered using pneumatic actuators.

Preferably, the vertical hammer rods in deep contact with the machined wooden boards cut the side surfaces using a cutting portion formed at their lower section.

Preferably, after the surface and deep impact machining using hammer rods, the machined wooden boards are subjected to a cleaning operation using the brush cleaning assembly and the pneumatic cleaning assembly.

Due to loosely seating the vertical hammer rods in the guide plate openings and equipping these vertical hammer rods with retaining elements for contacting the guide plate, it was possible to raise the guide plate together with the vertical hammer rods to a fixed height. Dynamic lowering of this guide plate with actuators allows for free gravitational falling of these vertical hammer rods from the fixed height, ensuring that the accumulated potential gravitational energy of each hammer rod is transferred to the machined wooden board. By selecting the fall height of the vertical hammer rods of known weight, the energy of their impact contact with the machined wooden boards can easily be determined. Proper selection of the impact contact energy allows for knocking out loose knots and cleaning other defects of the machined wooden boards without causing significant damage to their surface.

The use of pneumatic actuators for driving the guide plate allows for a suitable speed of displacement, allowing for dynamic lowering of this guide plate and thus a gravitational fall of the vertical hammer rods. In addition, the pneumatic actuators ensure greater cleanliness and lack of possible leakage of hydraulic fluids adversely affecting the surfaces of the machined wooden boards.

The shaping of the retaining elements of the vertical hammer rods above the guide plate allows them to be lifted when displacing this plate upwards and to fall freely by gravity when lowering this plate.

The shaping of the cutting portion above the lower face portion of the vertical hammer rods allows for a more efficient machining of the side surfaces of deep decrements in the machined wooden boards.

### BRIEF DESCRIPTION OF THE DRAWINGS

The subject of the invention is presented in the drawing by means of embodiments, wherein:
Figure 1 shows a machining device for removing surface and deep defects in a longitudinal section with a view of working mechanisms;
Figure 2 shows the device of Figure 1 in an axonometric view, without an upper portion of the support structure and without supporting and guiding members for vertical hammer rods;
Figure 3a shows a section of the machining device with the machined wooden boards, with the guide plate lowered, and with the vertical hammer rods lying on the upper surface of the machined wooden board;
Figure 3b shows the same section of the device as in Figure 3a, with the machined wooden boards, with the guide plate lowered, and with the vertical hammer rods lying on the upper surface of the machined wooden board, and with a part of the vertical hammer rods passing through the hole left after the partially spoiled knot;
Figure 3c shows the same section of the device, with the machined wooden boards as in Figure 3a, in the further working sequence, with the guide plate lowered and with the vertical hammer rods lying on the upper surface of the machined wooden board, with a visible machined hole left after the partially spoiled knot;
Figure 4 shows an enlarged and limited section of the device of Figure 3a, with the guide plate lowered and with the vertical hammer rods lying on the upper surface of the machined wooden board;
Figure 5a shows a straight hammer rod;
Figure 5b shows a hammer rod with a guiding portion;
Figure 5c shows a hammer rod with a cutting portion shaped on the guiding portion;
Figure 6 shows, in a schematic plan view, a production line for machining of wooden boards with two machining devices according to the invention, with a cutting assembly for giving to these semi-finished products desired dimensions of thickness, width and length;
Figure 7 shows, in a schematic plan view, an alternative solution for a production line for machining of wooden boards with two machining devices according to the invention, without a cutting assembly;
Figure 8a, Figure 8b, Figure 8c, Figure 8d illustrate various exemplary defects of the machined wooden boards for removal by means of the device according to the invention;

### EMBODIMENTS OF THE INVENTION

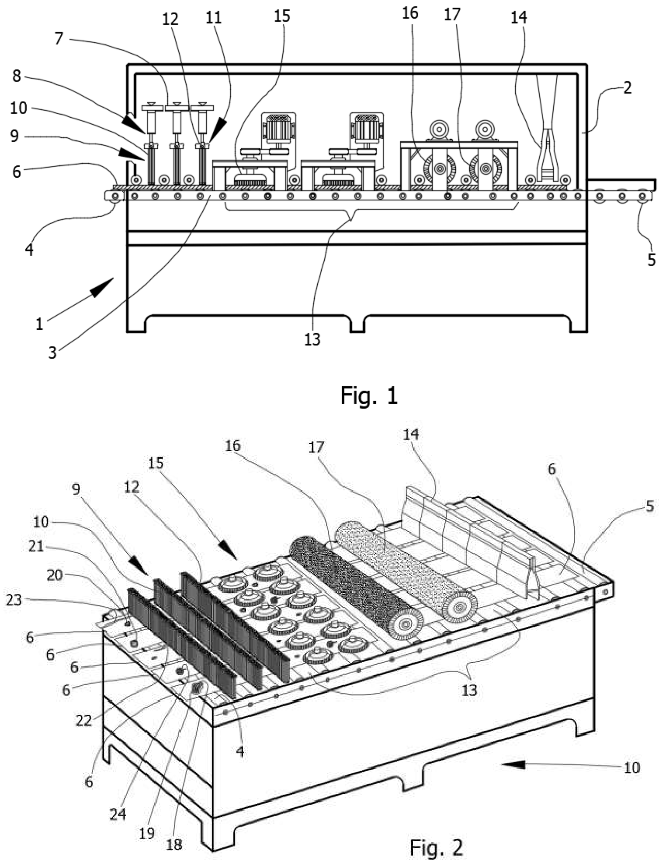

As depicted in Figure 1, machining device 1 for removing surface and deep defects has a support structure 2 on which a transport assembly 3 with transport rollers 4, 5 for horizontal displacement of the machined wooden boards 6 is supported. Above the transport assembly 3, an upper beam 7 is attached to the support structure 2, to which a hammer assembly 9 with three rows of vertical hammer rods 10, which are loosely seated in the guide plate 11, with the possibility of vertical displacement, is suspended by means of pneumatic actuators 8.

The vertical hammer rods 10 have retaining elements 12 for contacting the guide plate 11. When lifting these hammer rods 10 to a fixed height, the retaining elements 12 rest on the upper side of the guide plate. When the guide plate 11 is lowered by the pneumatic actuators 8 to the lowest position, the hammer rods 10 use the accumulated potential energy by hitting the surface or entering the recesses or penetrating the holes of the machined wooden boards 6. In this position, only those retaining elements 12, whose hammer rods 10 penetrated the holes of the machined wooden boards 6, rest on the guide plate 11. Behind the hammer assembly 9, in the direction of displacement of the machined wooden boards 6, a brush cleaning assembly 13 is positioned, followed by a pneumatic cleaning assembly 14.

The brush cleaning assembly 13 consists of two rows of wire brushes 15, rotating in axes perpendicular to the machined surface of the machined wooden boards 6, performing oscillating planetary movements. Behind these wire brushes 15, in the direction of displacement of the machined wooden boards, there is a pulling-and-tearing wire roller brush 16, performing corotating or counter-rotating movement around an axis parallel to the machined surface of the machined wooden boards 6, and behind this pulling-and-tearing wire roller brush 16, there is a cleaning roller brush 17, also performing a rotational movement around an axis parallel to the machined surface of the machined wooden boards 6.

The pneumatic cleaning assembly 14 positioned downstream of the cleaning roller brush 17 is in the form of a slot nozzle. The compressed air stream flowing out of the pneumatic cleaning assembly 14 removes residual impurities after performed mechanical surface and deep machining operations.

In order to better illustrate the arrangement of the cleaning elements and assemblies, Figure 2 shows the machining device 1 without the upper portion of the supporting structure 2 and without the supporting, guiding and driving elements. The machined wooden boards 6 with various surface and deep defects are moved on the transport rollers 4, 5. Various defects, such as: a partially spoiled knot 18 with a content of decay 19, a healthy knot 20, a knot hole 21, an internal crack 22, a frontal crack 23 and a burl 24, are shown on the surfaces of the machined wooden boards 6.

The hammer assembly 9 with three rows of vertical hammer rods 10 is positioned above the machined wooden boards 6. Each vertical hammer rod has the retaining elements 12 formed in the upper face portion. Behind the hammer assembly 9, the brush cleaning assembly 13 with two rows of the wire brushes 15, a pulling-and-tearing wire roller brush 16 and the cleaning roller brush 17 are positioned, and the pneumatic cleaning assembly 14 is positioned behind the brush cleaning assembly 13.

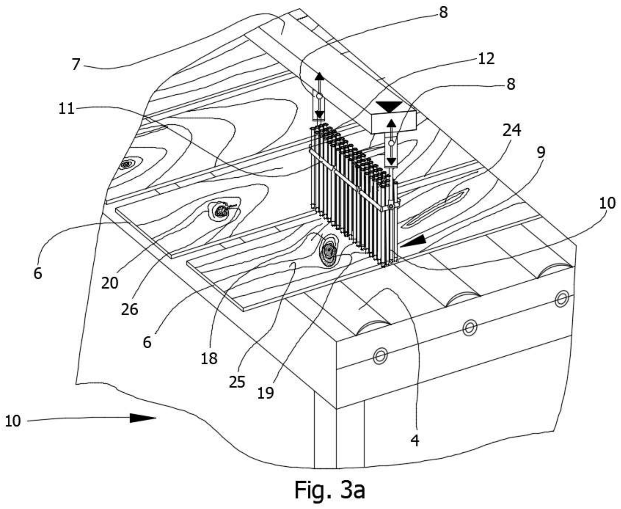

As shown in Figure 3a, the guide plate 11 of the hammer assembly 9 of the machining device 1 was shown in the position lowered by the pneumatic actuators 8 to the lowest position. In the guide plate 11, the hammer rods 10 terminated with the annular abutment members 12 in the upper face portion are arranged. The pneumatic actuators 8 are attached to the upper beam 7 on one side and to the guide plate 11 on the other.

Since there are no recesses or any surface defects in the area of the machined wooden board 6 under the vertical hammer rods 10 shown in Figure 3a, these vertical hammer rods 10 rest on its upper surface 25. This exemplary machined wooden board 6 has defects in the form of the partially spoiled knot 18 with some decay 19 and the burl24. On the adjacent wooden board 6 there is a defect in the form of the healthy knot 20 with an internal crack 26.

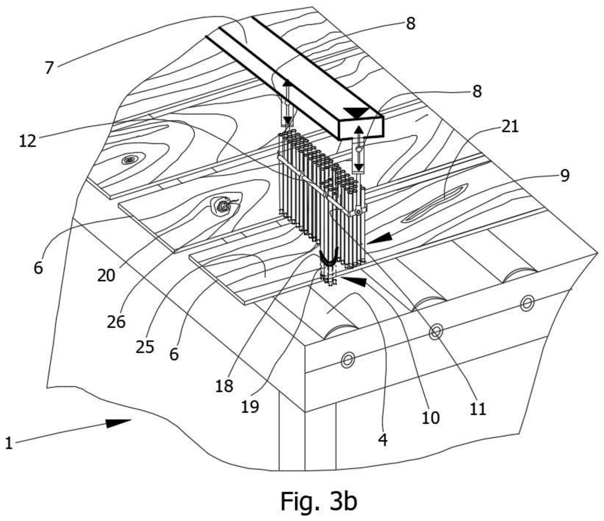

Figure 3b shows the same section of the machining device 1 with the machined wooden board 6 as in Figure 3a. In this position, the guide plate 11 is lowered by the pneumatic actuators 8. In the position shown, the area of the machined wooden board 6 with the partially spoiled knot 18 with the decay 19 was displaced on the transport rollers 4 to the area of operation of the hammer assembly 9 with the vertical hammer rods 10. The hammer rods 10, falling under their own weight, remove the decay 19 and other loose particles from the partially spoiled knot 18, passing locally through the machined wooden board 6. These hammer rods 10 that have passed through the machined wooden board 6 rest on the upper surface of the guide plate 11 with ring-shaped retaining elements 12.

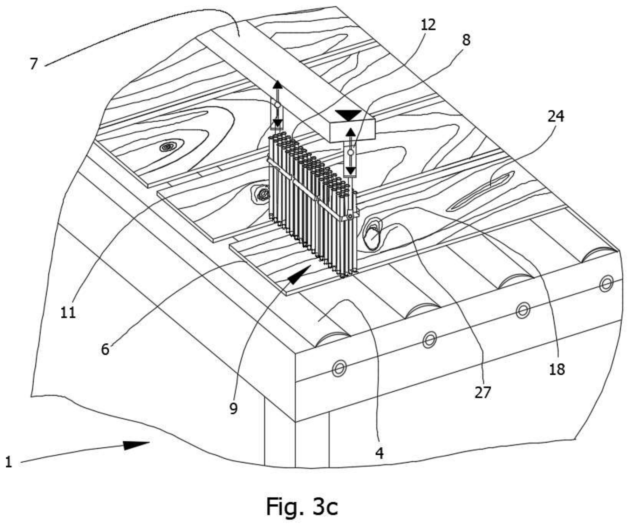

Figure 3c shows the same section of the machining device 1 with the machined wooden board 6 as in Figure 3a. In the position shown, the area of the machined wooden board 6 with a punched hole 27 in the area of the partially spoiled knot 18 and with the burl 24 was displaced on the transport rollers 4 outside the area of operation of the hammer assembly 9. In the absence of deep defects, the hammer rods 10 rest on the upper surface 25 of the machined wooden board 6. In this position, although the pneumatic actuators 8 have lowered the guide plate 11 to the lowest position, the ring-shaped retaining elements 12 do not rest on the upper surface of this guide plate 11. In the absence of deep defects, the falling height of the hammer rods is correspondingly lower, and therefore the possible potential energy of the hammer rods is not used. In this case, the kinetic energy of the accelerated hammer rods 10 slightly squeezes the upper surface 25 of the machined wooden board 6 without causing damage.

As shown in Figure 3a, Figure 3b and Figure 3c, a small number of installed hammer rods 10 is involved in the operation of removing of surface and deep defects in the machined wooden boards 6, wherein, during each sequence, hammer rods 10 are lifted by the guide plate 11 and, after dynamically lowering the guide plate 11, the hammer rods, falling freely, hit the upper surface of the machined wooden board 6. Since the arrangement of the wood defects in terms of position and shape is different for the individual machined wooden boards 6, therefore hammer rods 10 are installed in a curtain arrangement covering the entire working width of the machining device 1, although only hammer rods 10 covering exclusively the width of a single machined wooden board 6 are shown in Figure 3a, Figure 3b and Figure 3c for simplicity of the drawing.

The contact of the hammer rods 10 with the machined wooden boards 6 occurs during their free gravitational fall. Pneumatic actuators 8 attached to the upper beam 7 perform reciprocating movements in the vertical axis at a given frequency, raising and lowering the guide plate 11 with hammer rods 10. Falling hammer rods 10 hit the upper surface 25 of the wooden board 6 under the influence of their weight, penetrating various types of cracks and checks and knocking out loose or partially spoiled knots.

The machined wooden boards 6, after moving behind the hammer assembly 9, are subjected to brushing by means of the brush cleaning assembly 13, as shown in Figure 1 and Figure 2. The first brushing step uses the wire brushes 15, rotating in axes perpendicular to the machined upper surface 25 with additional oscillating planetary movements. In this step, the upper surface 25 and smaller recesses are cleaned of residues of loose fibers and non-structural substances of the wood. In a further brushing step, the machined wooden boards 6 are subjected to the action of the pulling-and-tearing wire roller brush 16, performing a rotational movement around an axis parallel to the upper surface 25 of the machined wooden boards 6. In this step, residues of loose wood particles within the checks, holes and other hidden and revealed defects remaining after the previous brushing step are finally removed. In the final brushing step, the machined wooden boards 6 are cleaned with the cleaning roller brush 17, also performing a rotational movement around an axis parallel to the upper surface 25. In this final brushing step, impurities formed in the previous steps are removed. The brushing step using the brush cleaning assembly 13 is followed by a step of removing leftover residues from previous operations by means of a compressed air jet directed by the slot nozzle of the pneumatic cleaning assembly 14.

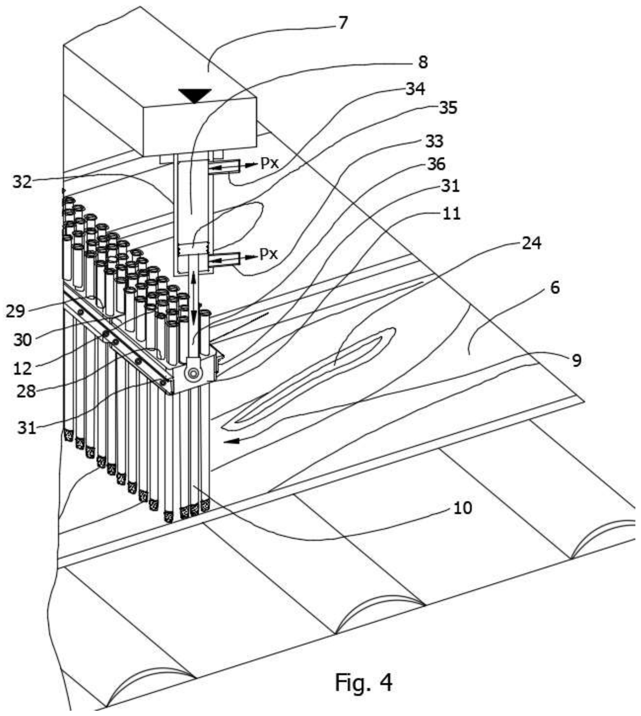

As shown in Figure 4, the vertical hammer rods 10 of the hammer assembly 9 are loosely seated in the guide plate 11 openings 28, suspended from the upper beam 7 by actuators 8. The guide plate 11 consists of segments 29, 30 connected on both sides by beams 31. The pneumatic actuator 8 has a cylinder 32 attached to the upper beam 7 and inlet ports 33, 34 supplying air under pressure Px. A piston 35 of the pneumatic actuator 8 is connected to a piston rod 36 which is coupled to the guide plate 11. The hammer rods 10 are loosely seated in the guide plate 11 openings 28, with the possibility of sliding axial displacement. Retaining elements 12 in the form of rings are formed in the upper face portion of each vertical hammer rod 10, wherein the diameter of each such retaining element 12 is larger than the diameter of the openings 28 in the guide plate 11, thereby allowing said retaining elements 12 to come into contact with the upper surface thereof.

By alternately cyclically applying the pressure Px to the lower port 33 and to the upper port 34, the piston rod 36 of the actuator 8 performs a reciprocating movement, thus causing reciprocating displacement of the guide plate 11 and lifting of the hammer rods 10. By rapidly lowering the guide plate 11, at a speed greater than the speed of gravitational fall of the hammer rods 10, a free gravitational fall of these hammer rods 10 and conversion of their potential energy into kinetic energy used to remove surface and deep defects of the machined wooden boards 6, is possible.

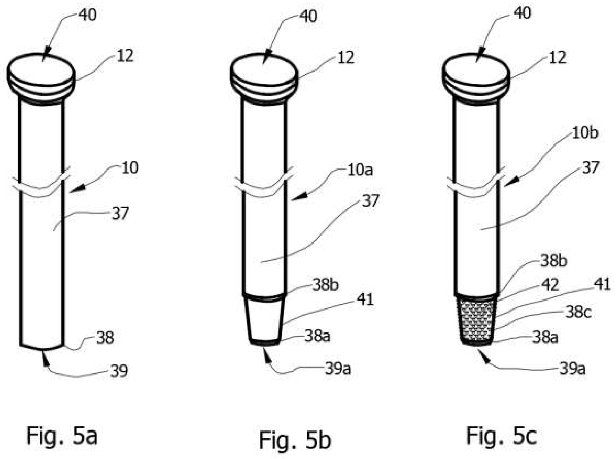

The vertical hammer rod 10 shown in Figure 5a has a cylindrical guiding portion 37, a cutting portion 38 formed around the circumference of a lower face portion 39, and the ring-shaped retaining element 12 is formed in an upper face portion 40.

In another embodiment shown in Figure 5b, the vertical hammer rod 10a has a tapered guiding surface 41 with a lower cutting portion 38a formed around the circumference of a lower face portion 39a and with an upper cutting portion 38b formed at the connection of the tapered guiding surface 41 and the cylindrical guiding portion 37. The ring-shaped retaining element 12 is formed in the upper face portion 40, as in Figure 5a.

As shown in Figure 5c in a further embodiment, the vertical hammer rod 10b has a rasp-cutting portion 38c in the form of rasp teeth 42 notched in the tapered guiding surface 41, between the lower cutting portion 38a of the lower face portion 39a and the upper cutting portion 38b formed at the connection of the tapered guiding surface 41 and the cylindrical guiding portion 37. The retaining elements 12 formed in the upper face portion 40 of each of the vertical hammer rods 10, 10a, 10b, shown in Figure 5a, Figure 5b or Figure 5c, have the same shapes.

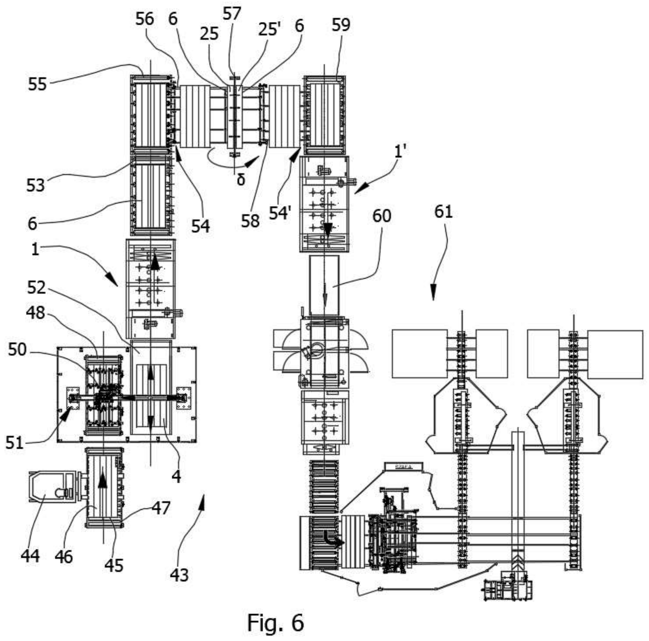

As shown in Figure 6, the machining device 1 is positioned in a production line 43 for machining of wooden boards 6. Packages 45 with semi-finished products 46 of the machined wooden boards 6 are transported by a forklift 44 to the production line 43 performing surface and deep machining. The semi-finished products 46 are laid on the surface of a roll feeder 47. The roll feeder 47 then transports the packages 45 to a scissor lift 48, from which a cross-bar 50 of a manipulator 51 takes the machined wooden boards 6 layer by layer, and places them on a belt conveyor 52. From the belt conveyor 52, a layer of machined wooden boards 6 is transported to the machining device 1, where a unilateral surface and deep machining is performed, as shown in Figure 1 and Figure 2. From the machining device 1, the unilaterally machined wooden boards 6 are received by a roll feeder 53 and transported to a cross-transport zone 54 consisting of a roll feeder 55 and a transverse belt conveyor 56. In the cross-transport zone 54, there is a change in the direction of movement of the semi-finished products from the longitudinal to the transverse, performed by the transverse belt conveyor 56, which transports the semi-finished products to a pocket turntable 57 performing a 180° rotation around a horizontal axis δ with the machined wooden board 6, changing position thereof so that the unmachined surface of the machined wooden board 6 is facing upwards. After performing such a semi-rotation around the horizontal axis δ, the previously machined upper surface 25 of the machined wooden board 6 becomes invisible and the unmachined lower surface 25' is on the visible side. This change in the position of the surface of the processed wooden boards 6 is necessary in order to perform surface and deep machining on both sides.

In a next cross-transport zone 54', a second transverse belt conveyor 58 moves the machined wooden boards 6 to a longitudinal roll feeder 59, which transports the machined wooden boards 6 to a machining device 1', in which their unmachined lower surface 25', currently facing upwards, is subjected to surface and deep machining in the same way as in the machining device 1. From the machining device 1', the wooden boards 6 machined on both sides are received by a belt conveyor 60. Subsequently, such wooden boards 6 machined on both sides are transferred to a machine assembly 61 for giving them the desired dimensions of thickness, width and length.

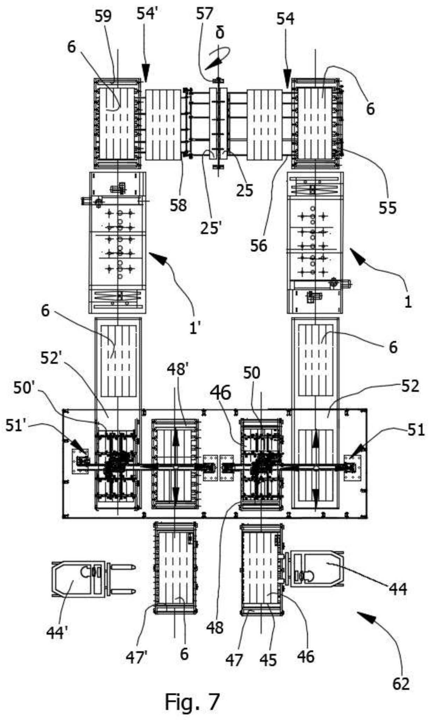

In an alternative embodiment, such as in Figure 7, a production line 62 does not include the machine assembly 61, such as in Figure 6. As shown in Figure 7, the forklift 44 carries the packages 45 with the semi-finished products 46 of the machined wooden boards 6. The semi-finished products 46 are laid on the surface of the roll feeder 47. The roll feeder 47 then transports the packages 45 to the scissor lift 48, from which the cross-bar 50 of the manipulator 51 takes the machined wooden boards 6 layer by layer, and places them on the belt conveyor 52. From the belt conveyor 52, a layer of machined wooden boards 6 is transported to the machining device 1, where a unilateral surface and deep machining is performed, as shown in Figure 1 and Figure 2. From the machining device 1, the unilaterally machined wooden boards 6 are transported to a cross-transport zone 54 consisting of the roll feeder 55 and the transverse belt conveyor 56. In the cross-transport zone 54, there is a change in the direction of movement of the semi-finished products from the longitudinal to the transverse, performed by the transverse belt conveyor 56, which transports the semi-finished products to the pocket turntable 57 performing a 180° rotation around the horizontal axis δ with the machined wooden board 6, changing its position so that the unmachined surface of the machined wooden board 6 is facing upwards. After performing such a 180° rotation around the horizontal axis δ, the previously machined upper surface 25 of the machined wooden board 6 becomes invisible and the unmachined lower surface 25' is placed on the visible side. Such a change in the position of the surfaces of the machined wooden boards 6 is necessary in order to perform surface and deep machining on both sides.

In the next cross-transport zone 54', the second transverse belt conveyor 58 moves the machined wooden boards 6 to the longitudinal roll feeder 59, which transports the machined wooden boards 6 to the machining device 1', in which their unmachined lower surface 25', currently facing upwards, is subjected to surface and deep machining in the same way as in the machining device 1. From the machining device 1', the both sides machined wooden boards 6 are received by a belt conveyor 52', from which a cross-bar 50' of a manipulator 51' lays these machined wooden boards 6 on a scissor lift 48'. Further, the both sides machined wooden boards 6 are received by a forklift 44' of a roll feeder 47'.

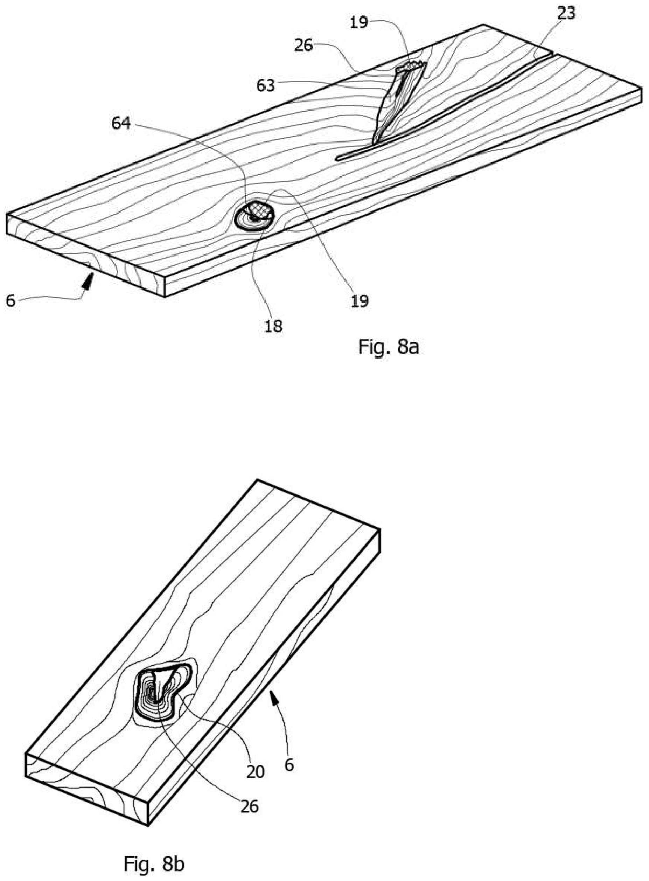

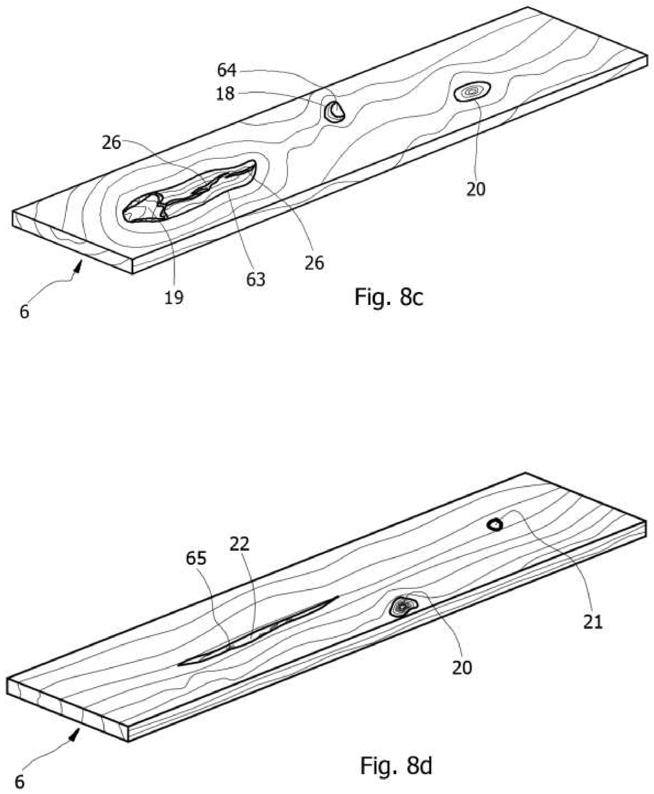

The device and method according to the invention enable the machining of various types of surface and deep defects occurring in the machined wooden boards 6. Such exemplary defects are shown in Figure 8a, Figure 8b, Figure 8c, Figure 8d.

The defects shown in Figure 8a are the frontal crack 23, an elongated knot 63 with the internal crack 26 and the decay 19 area, and the partially spoiled knot 18 with the decay 19 area and a wood decrement area 64.

The defects shown in Figure 8b show defects in the anatomical structure of wood in the form of the healthy knot 20 with an area of the significant internal crack 26.

Figure 8c shows defects in the form of the healthy knot 20, the partially spoiled knot 18 with a wood decrement 64 and the elongated knot 63 with the decay 19 area and the internal cracks 26 in the knot.

Figure 8d shows the internal crack 22 with loose fibers 65 and the healthy knot 20 and the knot hole 21.

### List of references:

1, 1' machining device
2 support structure
3 transport assembly
4, 5 transport roller
6 wooden board
7 upper beam
8 pneumatic actuator
9 hammer assembly
10, 10a, 10b hammer rod
11 guide plate
12 retaining element
13 brush cleaning assembly
14 pneumatic cleaning assembly
15 wire brush
16 wire roller brush
17 cleaning roller brush
18 partially spoiled knot
19 decay
20 healthy knot
21 knot hole
22 internal crack
23 frontal crack
24 burl
25 upper surface
25' lower surface
26 internal crack
27 punched hole in the area of a partially spoiled knot
28 guide plate opening
29, 30 guide plate segment
31 beam
32 cylinder
33, 34 port
35 piston
36 piston rod
37 cylindrical guiding portion
38, 38a, 38b, 38c cutting portion
39, 39a 39b lower face portion
40 upper face portion
41 tapered guiding surface
42 rasp teeth
43 production line
44, 44' forklift
45 packages
46 semi-finished products
47, 47' roll feeder
48, 48' scissor lift
50, 50' cross-bar
51, 51' manipulator
52 belt conveyor
53 roll feeder
54, 54' cross-transport zone
55 roll feeder
56 transverse belt conveyor
57 pocket turntable
58 second transverse belt conveyor
59 longitudinal roll feeder
60 belt conveyor
61 machine assembly
62 production line
63 elongated knot
64 wood decrement area
65 loose fibers
Px pressure
δ horizontal axis

## Claims

1. A machining device (1) for removing surface and deep defects in wooden boards (6) comprising:
- a support structure (2);
- a transport assembly (3) for horizontal displacement of the machined wooden boards (6) on transport rollers (4, 5);
- a guide plate (11) with openings (28) disposed under an upper beam (7) of the support structure (2) above the transport assembly (3);
- a hammer assembly (9) with at least one row of vertical hammer rods (10, 10a, 10b) loosely seated in the guide plate (11) openings (28);
- actuators (8) for displacing the vertical hammer rods (10, 10a, 10b);
- retaining elements (12) formed on the vertical hammer rods (10, 10a, 10b);
- a brush cleaning assembly (13) positioned behind the hammer assembly (9), in the direction of displacement of the machined wooden boards (6);
- a pneumatic cleaning assembly (14) positioned behind the brush cleaning assembly (13), in the direction of displacement of the machined wooden boards (6);
**characterized in that**
the actuators (8) displacing the vertical hammer rods (10, 10a, 10b) are connected on one side to the guide plate (11) and on the other side to the upper beam (7) of the support structure (2), wherein
the vertical hammer rods (10, 10a, 10b) loosely seated in the guide plate (11) openings (28) have abutment members (12) for contacting the guide plate (11).

2. The device according to claim 1, **characterized in that** pneumatic actuators are used as the actuators (8).

3. The device according to any of the preceding claims, **characterized in that** the retaining elements (12) of the vertical hammer rods (10, 10a, 10b) are positioned above the guide plate (11).

4. The device according to any of the preceding claims, **characterized in that** the retaining elements (12) are formed in an upper face portion (40) of the vertical hammer rods (10, 10a, 10b).

5. The device according to any of the preceding claims, **characterized in that** the retaining elements (12) are formed as rings.

6. The device according to any of the preceding claims, **characterized in that** the vertical hammer rods (10, 10a, 10b) have a cutting portion (38, 38a, 38b, 38c) shaped in their lower portion.

7. The device according to claim 6, **characterized in that** the cutting portion (38, 38a) is formed around the circumference of a lower face portion (39, 39a).

8. The device according to claim 6, **characterized in that** the vertical hammer rod (10a, 10b) has a tapered guiding surface (41) with the lower cutting portion (38a) formed around the circumference of the lower face portion (39a) and with the upper cutting portion (38b) formed at the connection of the tapered guiding surface (41) and a cylindrical guiding portion (37).

9. The device according to claim 6, **characterized in that** the vertical hammer rod (10b) has a friction cutting portion (38c) in the form of rasp teeth (42) notched in the tapered guiding surface (41), between the lower cutting portion (38a) of the lower face portion (39a) and the upper cutting portion (38b) formed at the connection of the tapered guiding surface (41) and the cylindrical guiding portion (37).

10. A method of removing surface and deep defects in wooden boards (6), wherein:
- machined wooden boards (6) are displaced into the area of operation of a hammer assembly (9) with vertical hammer rods (10, 10a, 10b) loosely seated in a guide plate (11) openings (28), placed under an upper beam (7) of a support structure (2);
- vertical hammer rods (10, 10a, 10b) are driven into surface and deep impact contact with the machined wooden boards (6), after which the machined wooden boards (6) are displaced to an operation area of a brush cleaning assembly (13) and a pneumatic cleaning assembly (14), **characterized in that**
- the guide plate (11) is raised together with the vertical hammer rods (10, 10a, 10b), and then this guide plate (11) is lowered, allowing for gravitational falling of the vertical hammer rods (10, 10a, 10b) loosely seated therein for surface and deep impact contact with the machined wooden boards (6).

11. The method according to claim 10, **characterized in that** the guide plate (11) is raised and lowered using pneumatic actuators (8).

12. The method according to claim 10 or 11, **characterized in that** the vertical hammer rods (10, 10a, 10b) in deep contact with the machined wooden boards (6) cut the side surfaces using a cutting portion (38, 38a, 38b, 38c) formed at their lower section.

13. The method according to claims 10 to 12, **characterized in that** after the surface and deep impact machining with hammer rods (10, 10a, 10b), the machined wooden boards (6) are subjected to a cleaning operation using the brush cleaning assembly (13) and the pneumatic cleaning assembly (14).
